# EUROPEAN PATENT APPLICATION

(11) **EP 3 950 007 A1**
(43) Date of publication of application: **09.02.2022**
(21) Application number: 19921997.3
(22) Date of filing: 28.10.2019
(51) Int. Cl.: A61K 49/00

(54) **METHOD FOR PREPARING NANO DIAGNOSTIC AGENT CAPABLE OF SELECTIVE STAINING OF INFLAMMATORY ABNORMAL TISSUES OR TUMOR TISSUES**

(30) Priority: 27.03.2019 KR 20190035390
(71) Applicant: Tgel Bio Co., Ltd., Gangnam-gu, Seoul 30021 (KR)
(72) Inventor: OH, Keun Sang, Daejeon 34605 (KR); CHO, Han Weon, Seoul 05005 (KR); HWANG, Chang Soon, Incheon 21662 (KR); KIM, Sun Jong, Seoul 06009 (KR); CHUNG, Hye Sook, Seoul 06292 (KR); LEE, Hye Jin, Chungcheongbuk-do 28423 (KR)
(74) Representative: Botti, Mario
(86) International application number: PCT/KR2019/014225
(87) International publication number: WO 2020/197021

(57) **Abstract**

The present invention relates to a method for preparing a nano diagnostic agent capable of selective staining of abnormal inflammatory tissues or tumor tissues. The method for preparing a nano diagnostic agent, according to the present invention, is characterized by comprising: a step of forming a first mixture by mixing polysorbate 80, soybean oil, and a water-insoluble staining material; a step of forming a second mixture by mixing and then heating the first mixture and a triblock copolymer; a step of forming a nano platform solid by cooling the second mixture; a step of forming a third mixture by mixing a solvent with the nano platform solid; a step of removing impurities from the third mixture; and a step of forming a nano-composite by freeze-drying the third mixture from which the impurities are removed. Thereby, the present invention can increase the search efficiency of a disease lesion by being transmitted through micropores of a tumor through direct permeation and absorption so as to precisely distinguish normal tissues from abnormal tissues.

## Description

### [Technical Field]

The present invention relates to a method for preparing a nanodiagnostic agent capable of selectively staining abnormal inflammatory tissue or tumor tissue. More specifically, the present invention relates to a method for preparing a nanodiagnostic agent loaded with a staining material that can clearly distinguish abnormal inflammatory tissue or tumor tissue from normal tissue by specifically or selectively staining the tumor tissue using the microenvironment of the abnormally formed tissue.

### [Background Art]

Cells repeatedly divide and grow according to the cell cycle. If the suppression mechanism of cell division is insufficient or does not function for some reason, division and growth are repeated. As a result, an excessively proliferated cell mass is formed, which is called a tumor. Such a tumor is a tissue formed by excessive cell proliferation within a short time, and the density between cells is lower than that of a normal tissue.

In recent years, the explosive increase in the medical field of nanobiotechnology, which is a fusion of nanotechnology and biotechnology, functional nanoparticles capable of diagnosing or treating such tumors have been in the spotlight.

However, conventionally reported techniques include the preparation of a contrast agent that generally targets a receptor specifically expressed in tumor cells, detects a low pH around the tumor, or targets a receptor specifically expressed in tumor cells. However, there have been no reports of research on nano-based diagnostic agents that can be delivered through direct penetration and absorption through the micropores of the tumor to precisely distinguish between normal and abnormal tissues, thereby increasing the efficiency of searching for diseased sites.

### [Disclosure]

### [Technical Problem]

Accordingly, the present invention provides a method for preparing a nano-based diagnostic agent that can be delivered by direct penetration and absorption through the micropores of the tumor to precisely distinguish between normal and abnormal tissues, thereby increasing the efficiency of searching for diseased sites.

### [Technical Solution]

The objectives are achieved through a method for preparing a nanodiagnostic agent capable of selectively staining abnormal inflammatory tissues or tumor tissues. The method is comprised of the steps of: forming a first mixture by mixing polysorbate 80, soybean oil, and a water-insoluble staining material; forming a second mixture by mixing and then heating the first mixture and a triblock copolymer; forming a nanoplatform solid by cooling the second mixture; forming a third mixture by mixing a solvent with the nano platform solid; removing impurities from the third mixture; and forming a nano-composite by freeze-drying the third mixture from which the impurities are removed.

In one embodiment, the water-insoluble staining material is one staining material selected from the group consisting of curcumin, fluorescein isothiocyanate (FITC), Nile red, Ce6 (Chlorin e6) and PpIX (Protoporphyrin IX).

In one embodiment, the water-insoluble staining material is a water-insoluble staining material composite obtained through ionic bonding by dissolving a cationic first staining material and anionic second staining material in a solvent, respectively, and mixing the same.

In one embodiment, the first staining material is selected from the group consisting of indigo carmine, methylene blue, indocyanine green, toluidine blue and rhodamine B.

In one embodiment, the second staining material is selected from the group consisting of indocyanine green and methylene blue.

In one embodiment, the heating temperature in the step of forming the second mixture is 55 °C to 65 °C.

In one embodiment, the cooling temperature in the step of forming a nanoplatform solid is 0 °C to 10 °C.

In one embodiment, the solvent is tertiary distilled water.

Meanwhile, the objectives are achieved by a nano diagnostic agent capable of selective staining of inflammatory tissue, the agent including a nano-composite with a micelle structure, wherein the inner core of the micelle structure includes soybean oil and polysorbate 80 in which a water-insoluble staining material is dissolved, wherein the outer shell of the micelle structure includes a triple block copolymer for forming uniform particles without agglomeration due to precipitation of components included in the inner core in an aqueous solution, and wherein the nano-composite has an average diameter of 1 nm to 100 nm.

In another embodiment of the present invention, a diagnostic agent solution is one in which the diagnostic agent is dispersed in an injection solution or a buffer solution.

### [Advantageous Effects]

The method for manufacturing a nanodiagnostic agent according to an embodiment of the present invention may provide a nano-sized diagnostic agent for loading an active ingredient that is manufactured by a simple method based on a polymer melt phase transition without using any organic solvent or aqueous solution.

The method for manufacturing a nanodiagnostic agent according to an embodiment of the present invention may provide a nano-sized diagnostic agent for loading an active ingredient that has an ultrafine particle size in an aqueous solution by loading a water-insoluble staining material.

The method for manufacturing a nanodiagnostic agent according to an embodiment of the present invention may provide a nano-sized diagnostic agent for loading an active ingredient that has one or more spectroscopic characteristics according to the characteristics of the water-insoluble staining material.

The method for manufacturing a nanodiagnostic agent according to an embodiment of the present invention may provide a nano-sized diagnostic agent for loading an active ingredient that provides excellent tumor diagnostic functionality through selectively delivering a greater amount of the staining material to the tumor over time and staining the tumor when the staining material is administered intravenously.

The method for manufacturing a nanodiagnostic agent according to an embodiment of the present invention may provide a nano-sized diagnostic agent for loading an active ingredient that provides excellent tumor diagnostic functionality through delivering a greater amount of staining material to the tumor relative to other organs and staining the tumor when applied and washed locally to the tumor.

The method for manufacturing a nanodiagnostic agent according to an embodiment of the present invention may provide a nano-sized diagnostic agent for loading an active ingredient that provides a staining material with greater diagnostic or distinguishing functionality encouraging a larger amount of the staining material to be absorbed and permeated into the deformed abnormal tissue and tumor tissue , thereby staining the abnormal tissue and tumor tissue to a greater degree relative to general normal tissue, when evenly applied and washed locally to normal tissue, abnormal tissue deformed by inflammation, and tumor issue.

The method for manufacturing a nanodiagnostic agent according to an embodiment of the present invention may provide a nano-sized diagnostic agent for loading an active ingredient that may carry a plurality of staining materials.

### [Description of Drawings]

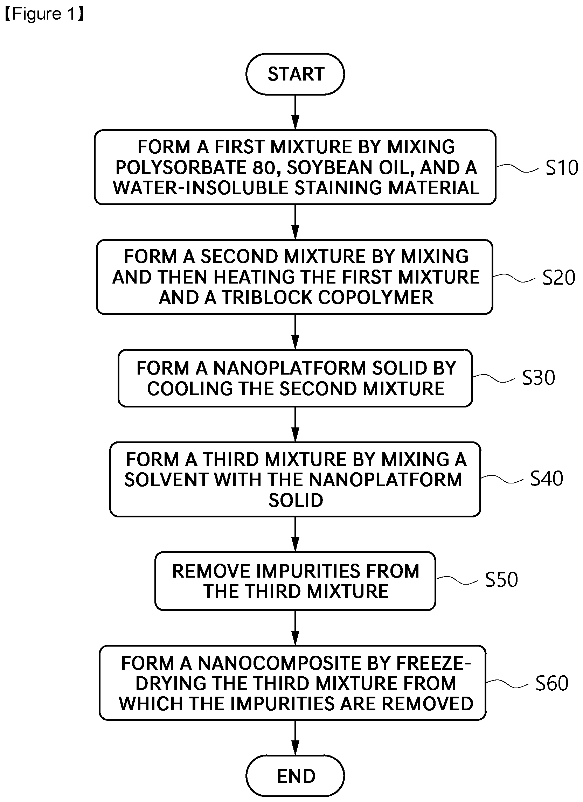

FIG. 1 is a flowchart depicting a method for manufacturing a diagnostic agent capable of selectively staining inflammatory abnormal tissue or tumor tissue according to an embodiment of the present invention.

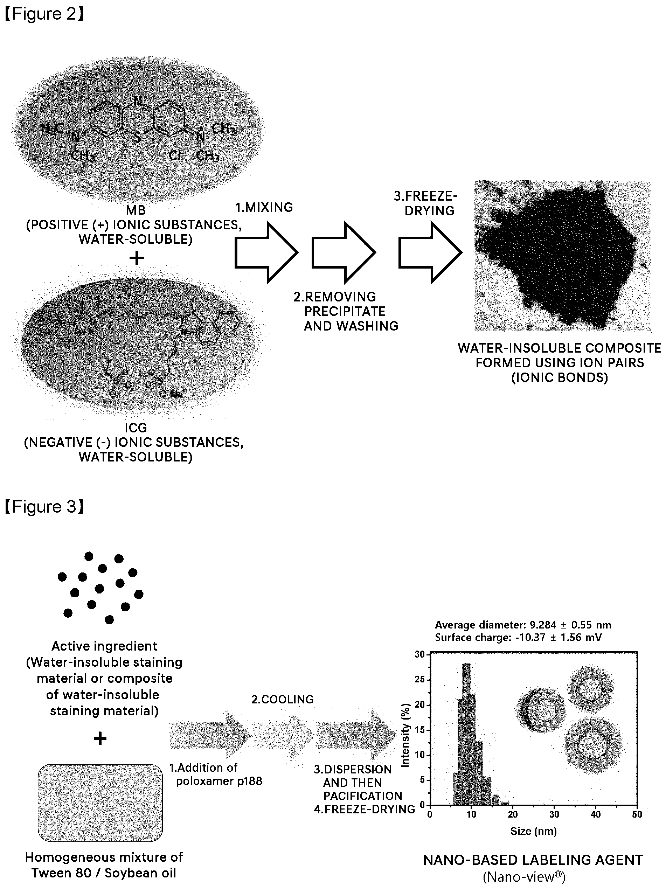

FIG. 2 is a schematic diagram depicting a method for forming a diagnostic agent loaded with a water-insoluble staining material or a water-insoluble composite according to an embodiment of the present invention.
FIG. 3 is a schematic diagram depicting a method for forming a water-insoluble composite in which staining materials ionized into cations and anions obtained by dissolving two water-soluble staining materials in an aqueous solution, respectively, are combined through ionic bonds according to an embodiment of the present invention.

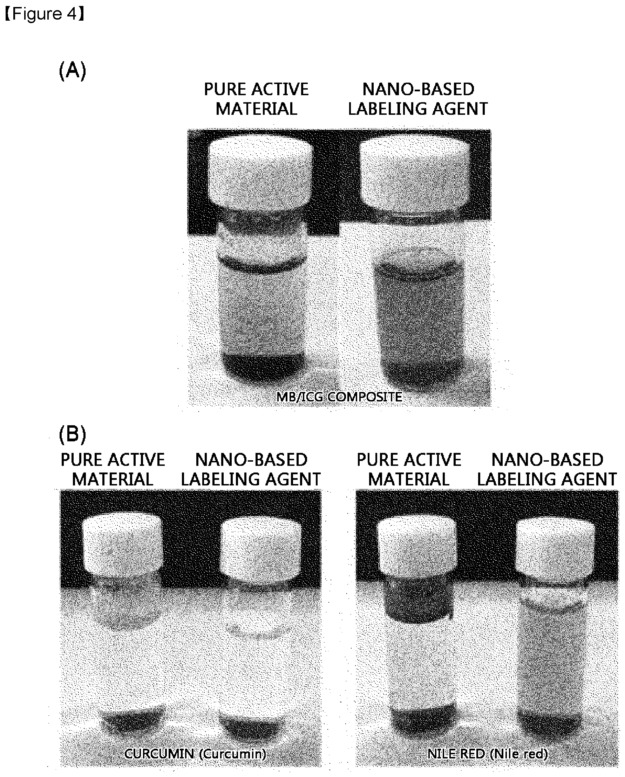

FIG. 4 is an image that depicts states in which (A) a diagnostic agent loaded with a water-insoluble composite in which cation and anion ionized staining materials are ionically bonded to each other according to an embodiment of the present invention and (B) a diagnostic agent loaded with a water-insoluble staining material is stably dispersed in the aqueous solution.

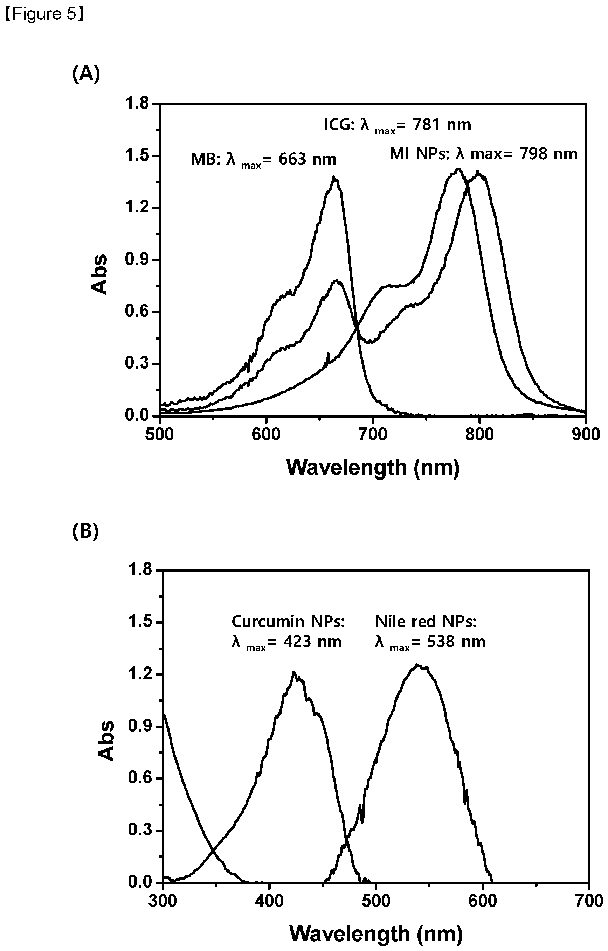

FIG. 5 is a graph that depicts the degree of absorbance obtained by measurement using ultraviolet-visible light spectroscopy for (A) a diagnostic agent loaded with a water-insoluble composite in which cation and anion ionized staining materials are ionically bonded to each other and (B) a diagnostic agent loaded with a water-insoluble staining material.

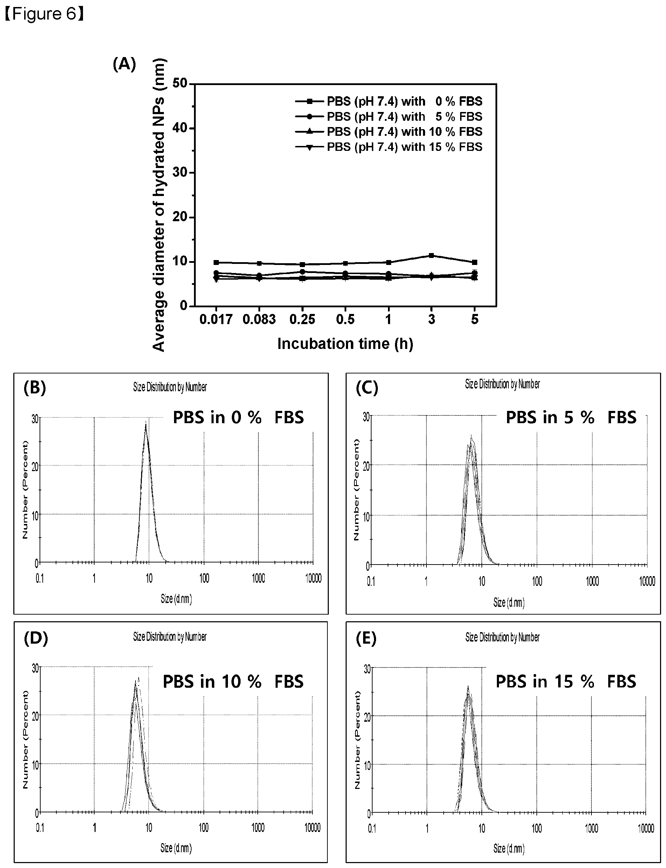

FIG. 6 is a graph that depicts the observation results for each time period using a particle analyzer to check particle stability according to the FBS content after dissolving the diagnostic agent loaded with the staining material according to an embodiment of the present invention in a buffer solution (pH 7.4) containing various concentrations of FBS.

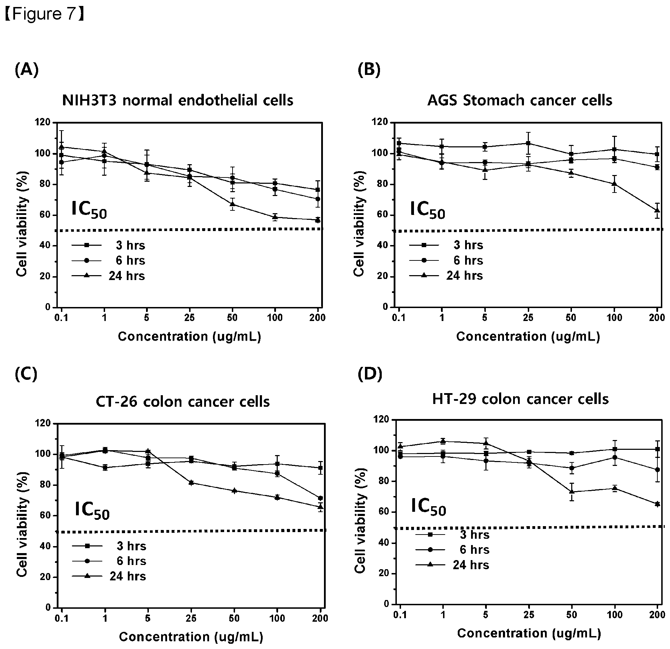

FIG. 7 is a graph that depicts the result of observing the degree of toxicity for each time period after treating various cells with a nano-based material that does not contain a staining material dispersed in the cell culture medium according to an embodiment of the present invention.

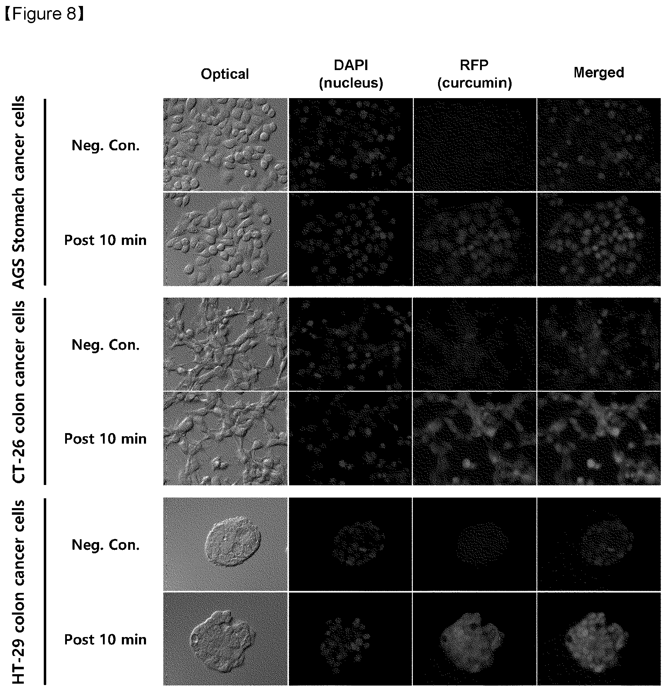

FIG. 8 is an image that depicts the degree of influx into cells measured for each time period using the fluorescence microscope after treating various tumor cells with a diagnostic agent loaded with a staining material dispersed in a cell culture medium according to an embodiment of the present invention.

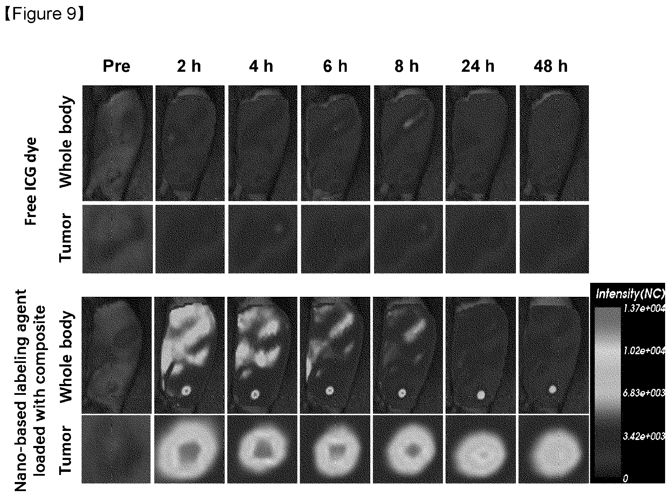

FIG. 9 is an image that depicts a real-time body distribution diagram and the degree of accumulation in tumors measured using a near-infrared fluorescence instrument after intravenous administration of a nano-based labeling agent loaded with a staining material dispersed in water for injection according to an embodiment of the present invention to a tumor-inducing mouse model implanted with tumor cells.

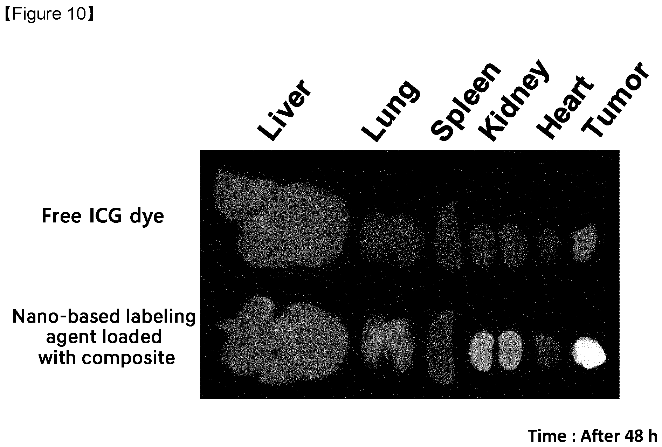

FIG. 10 is an image that depicts the degree of accumulation of the staining material measured by near-infrared fluorescence equipment for a main excised organ of each mouse obtained 48 hours after intravenous administration of a diagnostic agent loaded with a staining material dispersed in water for injection according to an embodiment of the present invention to a tumor-inducing mouse model implanted with tumor cells.

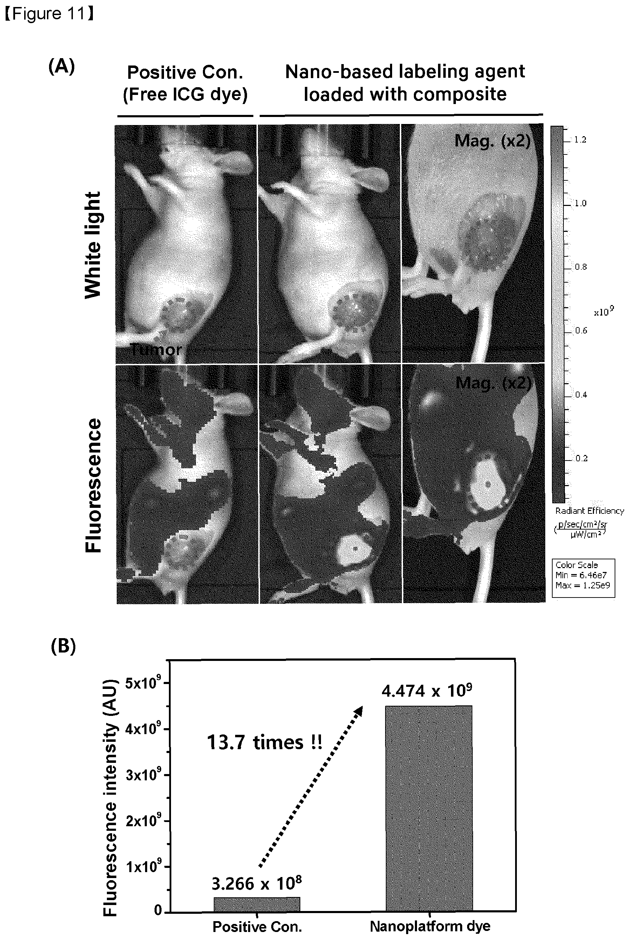

FIG. 11 is an image that depicts the degree of permeation and absorption of a material measured using a fluorescence instrument after direct application and washing of a diagnostic agent loaded with a staining material dispersed in water for injection according to an embodiment of the present invention to a tumor-induced mouse model tumor implanted with tumor cells.

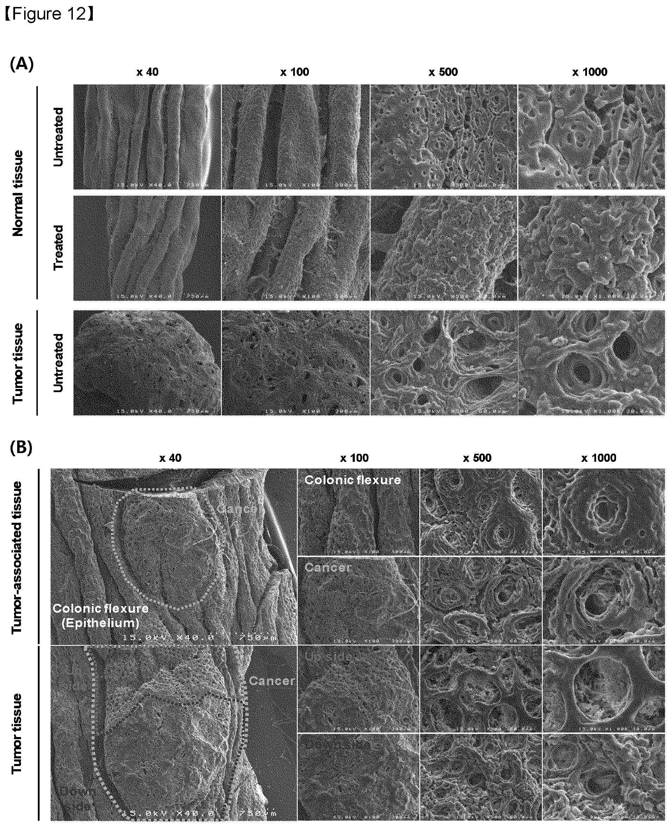

FIG. 12 is an image obtained by electron microscopy depicting (A) prior and (B) post direct application and washing of a diagnostic agent loaded with a staining material dispersed in water for injection according to an embodiment of the present invention to a tumor.

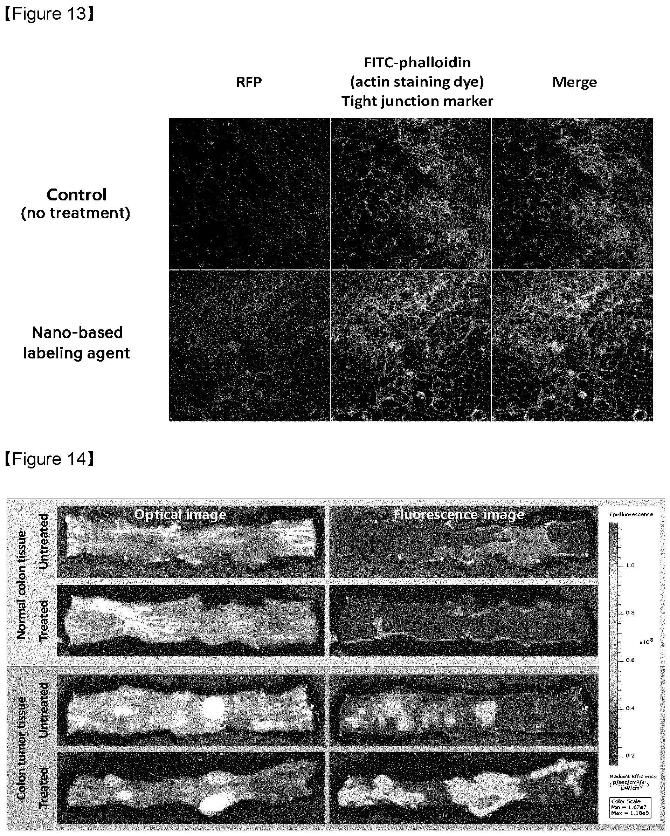

FIG. 13 is an image depicting measurements by the fluorescence microscope after treating the material in three-dimensionally cultured cells in order to confirm the tissue penetration mechanism of the diagnostic agent loaded with the staining material dispersed in the water for injection according to an embodiment of the present invention.
FIG. 14 is an image depicting measurements using a near-infrared fluorescence after direct application and washing of a diagnostic agent loaded with water-insoluble curcumin dispersed in water for injection according to an embodiment of the present invention to a tumor spontaneously formed in the colon due to inflammation.

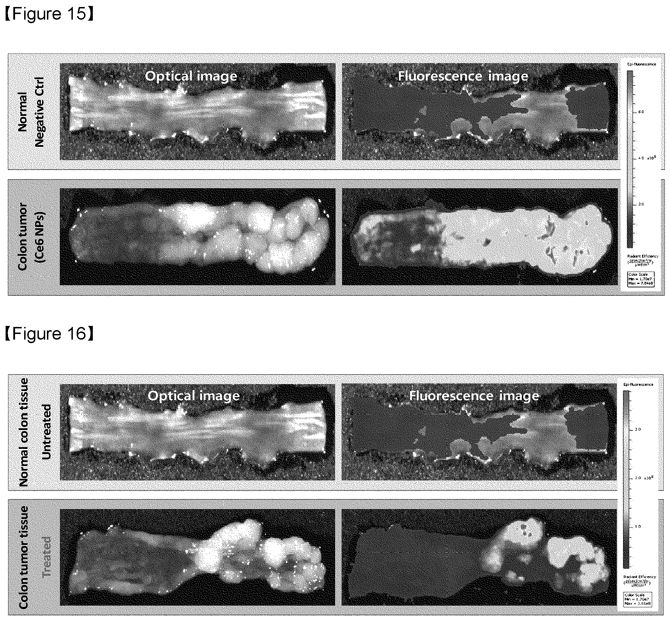

FIG. 15 is an image depicting measurements using a near-infrared fluorescence after direct application and washing of a diagnostic agent loaded with water-insoluble staining material, Nile red, dispersed in water for injection according to an embodiment of the present invention to a tumor spontaneously formed in the colon due to inflammation.
FIG. 16 is an image depicting measurements using a near-infrared fluorescence after direct application and washing of a diagnostic agent loaded with water-insoluble composite by ionic bonding dispersed in water for injection according to an embodiment of the present invention to a tumor spontaneously formed in the colon due to inflammation.

### [Modes of the Invention]

The terms used in the present application are only used to describe specific embodiments and are not intended to limit the present invention. Singular expressions include plural expressions unless the context clearly indicates otherwise. In the present application, terms such as "include" or "have" are intended to designate that features, elements, etc., described in the specifications exist, but these terms are not meant to indicate that one or more other features or elements may not exist or be added.

In addition, terms such as "first" and "second" used in the present application do not indicate an order, but are used to distinguish two components, and thus do not limit the two components. In particular, the terms "first mixture," "second mixture," and "third mixture" refer to mixtures including mixing elements presented before the appearance of each term, and in order to distinguish three mixtures, the terms "first," "second" and "third" are used.

The term "nano" used in the present application may refer to a size in a nanometer (nm) unit. For example, it may mean a size of 1 nm to 1,000 nm but is not limited thereto. In addition, the term "nanoparticle" used in the present application may refer to a particle having an average particle diameter in a nanometer (nm) unit, and for example, it may refer to a particle having an average particle diameter of 1 nm to 1,000 nm but is not limited thereto.

Unless otherwise defined, all terms used herein including technical or scientific terms have the same meanings as commonly used and understood by one of ordinary skill in the art to which the present invention belongs. Terms, such as those defined in a commonly used dictionary, should be interpreted as having a meaning contextually consistent with their context in relation to the related technology and should not be interpreted to have an ideal meaning or excessively formal significance unless explicitly defined in the present application.

Hereinafter, with reference to the accompanying drawings, a method for preparing a diagnostic agent capable of selective staining of an inflammatory abnormal tissue or tumor tissue according to the present invention is described. The scope of the method of preparing the diagnostic agent capable of selective staining is not limited by the accompanying drawings.

FIG. 1 is a flowchart depicting a method for manufacturing a diagnostic agent capable of selectively staining inflammatory abnormal tissue or tumor tissue according to an embodiment of the present invention. Furthermore, FIG. 2 is a schematic diagram depicting a method for forming a diagnostic agent loaded with a water-insoluble staining material or a water-insoluble composite according to an embodiment of the present invention.

As illustrated in FIGS. 1 and 2, first, polysorbate 80, soybean oil, and a water-insoluble staining material are mixed to form the first mixture (S 10).

In particular, the agent comprised of polysorbate 80 and soybean oil, which have not been used previously, distinguish the present invention, and, through this, fine nano-composite particles may have a size of 1 nm to 100 nm, which is described later in the present application.

Because polysorbate 80 has both hydrophilicity and hydrophobicity, it is widely used as a surfactant in pharmaceuticals and cosmetics. Polysorbate 80 also has stabilizing characteristics that help the particles to be stably dispersed in the emulsification process or a solubilizer that dissolves water-insoluble active ingredients. In addition, soybean oil does not mix with water at all, because of its water-insoluble property, and it is loaded into the particles through a hydrophobic bond with a water-insoluble active ingredient in the emulsification process.

In one embodiment of the present invention, the water-insoluble staining material generates an intrinsic absorption wavelength from a short wavelength to a long wavelength, or various materials capable of generating fluorescence by a specific light. In the present invention, it may be divided into a single material or a composite material.

As an example, the single material is a single staining material selected from the group consisting of curcumin, fluorescein isothiocyanate (FITC), Nile red, Ce6 (Chlorin e6) and PpIX (Protoporphyrin IX), but is not limited thereto.

Curcumin is a natural substance that helps in anti-inflammatory, antioxidant, anti-cancer, and blood circulation improvement, and it has a spectroscopic characteristic of an intrinsic absorbance of 340 nm to 534 nm. Nile Red is used as a water-insoluble staining material for staining cells or tissues, and spectroscopically, the maximum excitation wavelength is 549 nm and the maximum emission wavelength is 628 nm.

In another embodiment, the water-insoluble composite staining material may be one obtained through ionic bonding by dissolving a cationic first staining material and anionic second staining material in a solvent, respectively, and mixing the same.

Here, the first staining material and the second staining material do not specifically indicate an order as described above, and are terms used to refer to different kinds of staining materials. The first staining material is a water-soluble material that has cationic (basic) properties in aqueous solution, and the second staining material is a water-soluble material that has anionic (acidic) properties in aqueous solution.

For example, the first staining material may be selected from the group consisting of indigo carmine, methylene blue, indocyanine green, toluidine blue and rhodamine B, but is not limited thereto.

For example, the second staining material may be selected from the group consisting of indocyanine green and methylene blue, but is not limited thereto.

FIG. 3 is a schematic diagram depicting a method for forming a water-insoluble composite in which staining materials ionized into cations and anions obtained by dissolving two water-soluble staining materials in an aqueous solution, respectively, are combined through ionic bonds according to an embodiment of the present invention. As illustrated in FIG. 3, for example, the first staining material is dissolved in a solvent, the second staining material is dissolved in a solvent, and then the two solutions are mixed to form a composite. In addition, the unreacted solution of the supernatant is removed except for the result precipitated in the lower layer due to the formation of the composite, then redispersion and centrifugation are repeatedly performed with fresh tertiary distilled water to wash the unreacted material contained in the precipitate. Thereafter, freeze-drying is performed to obtain a staining material composite.

In other words, the present invention is to prepare a composite with water-insoluble properties by combining staining materials that originally have water-soluble properties but are ionized in an aqueous solution to have cation or anion properties with each other through ionic bonds.

Also, in one example, polysorbate 80 and soybean oil are preferably mixed in a weight ratio of 99:1 to 60:40. It is preferable to contain 0.01 to 0.1 parts by weight of the water-insoluble staining material, based on a total of 100 parts by weight of polysorbate 80 and soybean oil. Such a mixing ratio is an example of a mixing ratio capable of exhibiting the above-described effects intended by the present invention.

The first mixture, which may be formed based on the above descriptions, is a solution in which the aforementioned polysorbate 80, soybean oil, and a water-insoluble staining material are mixed and have transparent and uniform characteristics without foreign substances.

Then, the first mixture and the triple block copolymer are mixed and heated to form the second mixture (S20).

Here, the triple block copolymer, preferably Poloxamer 188, is a copolymer in which a hydrophilic group and a hydrophobic group are bonded. Poloxamer is a polymer of Poly(oxyethylene)-poly(oxypropylene)-poly(oxyethylene)[POE-POP-POE]. Poloxamer is solid at room temperature, which is soluble in water and ethanol. Poloxamer solution is liquid at low temperature, but viscosity increases as the temperature rises. When the concentration is high, it shows sol-gel thixotropy according to temperature. The poloxamer does not damage the mucosal cell membrane. Poloxamers 68, 127, 188, 237, 338, and 407 are commercially available. For example, poloxamer 188 indicates a poloxamer having a molecular weight of about 8350 as a compound of Formula 1 in which b is 30 and the sum of a and c is about 75.

In general, the introduction of the hydrophilic group is intended to provide the functionality to be uniformly and stably dispersed without aggregation of substances and active ingredients for a long time in an aqueous solution. In addition, the poloxamer solution is essential for conferring the critical functionality of delivering the substances to the desired disease site by increasing body circulation when administered to blood vessels. However, the present invention includes the introduction of a hydrophilic group so that it can permeate and be absorbed into cells or tissues rather than an agent administered through blood vessels. This introduction allows particles having an ultra-fine nano size (8 nm to 10 nm) to be stably and uniformly dispersed in an aqueous solution. Furthermore, the introduction of a hydrophobic group is essential for loading water-insoluble active ingredients (various drugs or staining material) having the same physical properties into the nanoplatform core.

Moreover, the heating temperature is preferably 55 °C to 65 °C. When the heating temperature is less than 55 °C, it is lower than the melting point of the tri-block copolymer, creating a problem in that it cannot be mixed with the first mixture. When it exceeds 65 °C, there is a problem in that the tri-block copolymer and the active ingredient decompose.

The second mixture, which may be formed based on the above descriptions, is obtained by adding a triblock copolymer to a solution in which the aforementioned polysorbate 80, soybean oil, and a water-insoluble staining material are mixed, which is also transparent and uniform.

Then, the second mixture is cooled to form the solid nano-platform (S30).

Here, the cooling temperature is preferably 0 °C to 10 °C. When the cooling temperature exceeds 10 °C, the second mixture is gradually cooled to lower the crystallization of the particles creating a problem in that the uniformity of the particle size is decreased.

The nano-platform solid may be formed based on the above descriptions. As described above, "nano" may mean a size of 1 nm to 1,000 nm. The nano-platform may be a carrier or vehicle on which the above-described water-insoluble staining material can be loaded. This may include a carrier such as a drug or a contrast agent that can be applied in the technical field to which the present invention pertains.

Then, the nano-platform solid is mixed with a solvent to form the third mixture (S40).

Here, the solvent is tertiary distilled water. This solvent dissolves the nanoplatform solids. The active ingredients or substances included therein are agglomerated, so that most of them do not cause residue and thus are uniformly and stably dispersed.

One of the features of the present invention is that no organic solvent is used. When an organic solvent is used, the formulation loaded with the active ingredient is destructed so that it cannot be used. In addition, even if the destruction does not occur, the step of removing the organic solvent must be necessarily included, as there is a possibility of inducing toxicity in the body due to the residual solvent.

When tertiary distilled water is used, there is no possibility of toxicity caused by solvents during the manufacturing process, so it has high biocompatibility. Further, it is different from the method (self-assembly emulsification) that uses the existing organic solvents that are widely used to manufacture liposome-based or polymer-based nanoplatforms.

The third mixture, which may be formed based on the above description, has the characteristics of being transparent and uniform.

Then, impurities are removed from the third mixture (S50).

The method for removing impurities is not particularly limited, and any method may be applied as long as it is a method applied in the technical field to which the present invention pertains. For example, impurities may be removed using a 0.2 µm to 1.2 µm filter, a 0.5 µm to 1.1 µm filter, a 0.6 µm to 1.0 µm filter, a 0.7 µm to 0.9 µm filter, or a 0.8 µm filter.

Then, the third mixture from which impurities have been removed is freeze-dried to form the nano-composite (S60).

Freeze-drying is a method of removing moisture and is a method of controlling the sublimation of solid water into a gas by lowering the atmospheric pressure after freezing the object. Here, although the specifics of freeze-drying are not presented, it is obvious that any freeze-drying method applicable in the technical field to which the present invention belongs can be applied.

The nano-composite may be formed in a solid form. The nano-composite has the unique color of the active ingredient and has the characteristic of maintaining unique spectroscopic properties.

Another embodiment of the present invention is made of such a nano-composite. Specifically, the present invention includes an organic light emitting dye and a nanoplatform for mounting the same. The present invention may provide a diagnostic agent capable of selectively staining abnormal inflammatory tissue or tumor tissue having an average diameter of 1 nm to 100 nm, preferably 1 nm to 90 nm, preferably 1 nm to 80 nm, preferably 1 nm to 70 nm, preferably 1 nm to 60 nm, preferably 1 nm to 50 nm, preferably 1 nm to 40 nm, preferably 1 nm to 30 nm, preferably 1 nm to 20 nm, preferably 3 nm to 15 nm, preferably 5 nm to 12 nm, preferably 7 nm to 10 nm, and most preferably 8 nm to 9 nm.

As described above, when the average diameter of the nano-composite is excessively large, for example, when it exceeds 100 nm, direct absorption in a tissue such as a tumor is difficult and thus cannot be applied to the intended application method of the present invention. That is, according to the present invention, as described above, the average diameter of the nano-composite is controlled to be ultra-fine to provide a nanoplatform optimized for the direct application method that could not be implemented in the nano-sized carrier described in some conventional literature.

When the diagnostic agent material does not have a nano size, it is an important factor to have an average diameter that falls within the above-mentioned range because the penetration and absorption rate into the tissue is not high. The above-mentioned size allows higher absorption and penetration into the tumor tissue relative to normal tissue so as to differentiate the tumor.

In addition, as described above, an essential condition of the material loaded on the nanoplatform is that it must have water-insoluble properties, and the effective water-insoluble material is hardly soluble unless an organic solvent such as ethanol, methanol, acetone, or DMSO is used. In addition, it is not mixed at all and floats or sinks in tertiary distilled water, so it is non-uniform.

Furthermore, another embodiment of the present invention may provide a diagnostic agent solution in which the aforementioned diagnostic agent is dispersed in an injection solution or a buffer solution. The diagnostic agent solution has a uniform and transparent character.

Accordingly, the present invention may provide a nano-based diagnostic agent that can be selectively delivered and stained only to abnormal tissues or tumors modified by inflammation.

In addition, when the diagnostic agent is applied on a whole area regardless of the specific site where the disease has occurred and then is washed with physiological saline, the permeation and absorption rate of the labeling agent is higher in abnormal and tumor tissues transformed by inflammation relative to normal tissues, thereby selectively staining the abnormal and tumor tissues to precisely stain (diagnose) the diseased area.

In addition, the diagnostic agent is uniformly dispersed in a solution suitable for the purpose (water for injection, buffer solution, distilled water, etc.), and then the mixture is administered through a blood vessel or applied directly to the tumor tissue, thereby precisely distinguishing between normal tissue and tumor tissue.

Here, as an example of water for injection, 0.5% to 1.3% NaCl solution, preferably 0.6% to 1.2% NaCl solution, preferably 0.7% to 1.1% NaCl solution, preferably 0.8% to 1.0% of NaCl solution, most preferably 0.9% NaCl solution may be used. Also, a 2% to 8% glucose solution, preferably a 3% to 7% glucose solution, preferably a 4% to 6% glucose solution, and most preferably a 5% glucose solution may be used.

Hereinafter, the present invention is described in more detail through Examples and Experimental Examples.

### Experimental Example 1. Preparation of a water-insoluble staining material composite using an ion-pair bond (ionic bond) of water-soluble methylene blue and indocyanine green

After completely dissolving 0.1 g of methylene blue and 0.1 g of indocyanine green, respectively in 20 mL of tertiary distilled water at room temperature, the indocyanine green aqueous solution was added to the methylene blue aqueous solution under stirring. The centrifugation was then performed to form a composite, and the unreacted solution of the supernatant was removed except for the formation precipitated in the lower layer. Then, redispersion and centrifugation were repeatedly performed with fresh tertiary distilled water. The unreacted material contained in the precipitate was washed. Thereafter, freeze-drying was performed to prepare a water-insoluble methylene blue/indocyanine green staining material composite for tissue staining.

### Experimental Example 2. Preparation of a nano-based labeling agent loaded with insoluble staining material using melt phase transition or polymer

In order to prepare a nano-based labeling agent loaded with a water-insoluble staining material using the melt phase transition of a polymer, 0.01 g of the water-insoluble staining material prepared in Experimental Example 1 was added to 0.6 g of soybean oil and Tween 80. The mixture was sufficiently stirred at room temperature to obtain a uniform and transparent state. Then, 0.4 g of F68 was added to the mixture, and the mixture was sufficiently stirred to prepare a uniform and transparent solution while heated to 60 °C and cooled to prepare a solid form. The prepared solid was dissolved in tertiary distilled water and filtered through a 0.8 µm filter to remove water-insoluble, aggregated staining material that was not loaded into the nano-based diagnostic agent. Furthermore, 0.2 µm filtration sterilization was performed according to the experimental schedule to be carried out later. The filtered solution was freeze-dried to finally obtain a diagnostic agent stably loaded with a staining material.

The diagnostic agent loaded with the staining material prepared in this way was easily dissolved in an aqueous solution. The average particle size was measured. The result of the measurement confirmed the particles to have a diameter of about 10 nm.

The versatility of the nano-agents loaded with curcumin and Nile red, which is water-insoluble regards intrinsic solubility in aqueous solution, as well as water-insoluble nano-composites obtained using ion-pair bonding (ionic bonding) of water-soluble staining materials in the same manner as described above was confirmed.

The results confirmed that not only the water-insolubility of the staining material composite, but also that various water-insoluble staining materials may be loaded in the nano agent and be effectively dispersed. Thus, the versatility is high because a variety of materials may be loaded instead of only specific materials.

### Experimental Example 3. Confirmation that the diagnostic agent loaded with the staining material is stably dispersed in the aqueous solution

In order to check whether the diagnostic agent loaded with the staining material of the present invention is stably dispersed in an aqueous solution, etc., the images of the pure active substance of the diagnostic agent loaded with the staining material prepared in Experimental Examples 1 and 2 and the diagnostic agent containing the same are dispersed in an aqueous solution are shown in FIG. 3.

FIG. 3 offers confirmation that each diagnostic agent was stably dispersed in the aqueous solution.

### Experimental Example 4. Spectroscopic characterization of diagnostic agent loaded with staining materials

Since a diagnostic agent loaded with staining material must be activated from the outside by a light source that matches the material's intrinsic wavelength, it is important to maintain the spectroscopic properties of the diagnostic agent. Therefore, the diagnostic agents loaded with the staining material obtained in Experimental Examples 1 and 2 were dispersed in tertiary distilled water, then the absorbance was checked using an ultraviolet-visible light spectrometer. The results are shown in FIG. 5.

As shown in FIGS. 5(A) and 5(B), it was confirmed that the intrinsic absorption wavelength was well maintained in accordance with the type of staining material loaded in the diagnostic agent.

### Experimental Example 5. Stability evaluation of diagnostic agent loaded with staining material in plasma

A diagnostic agent loaded with a staining material should be uniformly well dispersed in an aqueous solution and maintained without significant change in particle size for a certain period of time. In addition, in the case of delivery into the body, there is a high possibility of contact with plasma depending on the route of administration. Thus, in order to ensure the stability of the material to be delivered, consideration should be given to the maintenance of particle size even in the presence of fetal bovine serum (FBS).

Therefore, after dispersing the diagnostic agents loaded with the staining material prepared in Experimental Example 1 in an electrolyte solution (phosphate buffered saline, PBS, pH7.4) containing plasma of various concentrations, the change in particle size was checked using a particle size analyzer. The measurement results are illustrated in FIG. 6. As shown in FIG. 6, it was confirmed that the particle size of the diagnostic agent loaded with the staining material did not significantly increase in the electrolyte solution containing various plasma, and the initial particle size was well maintained, thereby ensuring stability in aqueous solution.

### Experimental Example 6. Toxicity Evaluation of diagnostic agent loaded with staining material in various cells

It is important to have low toxicity, because the diagnostic agent loaded with the staining material has no therapeutic function and has a function for staining simple cells or tissues. In order to evaluate the toxicity at the cellular level of the diagnostic agent loaded with the staining material prepared in Experimental Example 1, the change was checked in normal cells (NIH3T3) and in various tumor cells (AGS, CT-26 and HT-29) according to various concentrations and treatment times. The measurement results are illustrated in FIG. 7.

As shown in FIG. 7, it was confirmed that the agents had low toxicity at various concentrations and cell treatment times.

### Experimental Example 7. Evaluation of influx behavior of diagnostic agents loaded with staining material in tumor cells

Various tumor cells (AGS, CT-26 and HT-29) were used to evaluate the influx behavior of the diagnostic agent loaded with the staining material prepared in Experimental Example 1 into cells. After treatment for a certain period of time, an image was obtained using a fluorescence microscope. The results are shown in FIG. 8.

As shown in FIG. 8, it was confirmed that the diagnostic agent loaded with the staining material could be introduced into the cell within a short time, and furthermore, it was confirmed that it was delivered not only to the cytoplasm but also to the cell nucleus despite the short amount of time.

### Experimental Example 8. Evaluation of tumor accumulation (targeting) and body distribution behavior of a diagnostic agent loaded with staining material in a tumor-inducing animal model

The diagnostic agent loaded with the staining material to be provided through the present invention should effectively accumulate in the tumor tissue, and the delivery to the normal tissue should be low to minimize the side effects caused by the material. As a tumor-inducing animal model, SCC-7 (squamous cell carcinoma) tumor cell-line (1×10^6 pieces/head) was implanted subcutaneously in the thigh of a rat. About 10 days later, a group with well-formed 150 mm^3 tumors was used in the experiment. Then, the diagnostic agent prepared in Experimental Example 1 was administered through the tail vein of the tumor-inducing animal model. After systemic circulation, tumor accumulation (targeting) was measured over time. After final analysis and evaluation, major organs were extracted. The accumulation level of the nano-based agent containing the staining material was confirmed through real-time fluorescence analysis equipment (IVIS Spectrum In Vivo Imaging System, PerkinElmer) and ex vivo fluorescence analysis equipment (KODAK Image Station 4000MM Digital Imaging System, Bruker BioSpin), respectively. The results are shown in FIGS. 9 and 10, respectively.

As shown in FIG. 9, it was confirmed that the diagnostic agent loaded with the staining material administered through the tail vein of the tumor-inducing animal model was quickly accumulated into the tumor by the systemic circulation.

In addition, as shown in FIG. 10, it was confirmed that the agents were only minimally accumulated in normal organs but largely accumulated in tumors in the major organs removed after the evaluation.

Further, the material was also detected in the kidney, which confirms that the administered diagnostic agent loaded with the staining material is removed through the kidney. Thus, it was confirmed that the agent did not have a problem of unnecessary accumulation in the body, which can cause side effects. Thus, it was confirmed that the above-described basic conditions can be satisfied.

### Experimental Example 9. Evaluation of the possibility of tissue staining through direct application of a diagnostic agent loaded with staining material to the tumor of a tumor-inducing animal model

Drug development has been a goal in conventional domestic or foreign studies related to diagnosis or diagnosis/treatment using a nano-based platform. Thus, the route of delivery in the body is largely oral and parenteral. However, there is little or very little oral absorption rate in most cases. Therefore, a more efficient parenteral administration method is preferred. However, since the diagnostic agent loaded with the staining material provided by the present invention is not limitedly applied to the conventional method, it is necessary to evaluate the possibility of the direct application method to increase user convenience. The application method will be included in the method of using the material in the future.

In order to confirm these characteristics, tumor accumulation and distribution of major organs of the diagnostic agent loaded with the staining material administered through the tail vein of the animal were evaluated. Further, staining of tumor tissue by the permeation and absorption into the tumor was evaluated even when the material is directly applied to the tumor tissue.

The subcutaneous tissue of the tumor formed in the tumor-inducing animal model was removed. Then the diagnostic agent loaded with the staining material prepared in Experimental Example 2 was applied on the site. Then the site was washed with tertiary distilled water 2 to 3 times. Then changes in fluorescence expressed in tissues were taken with fluorescence imaging equipment (IVIS) Spectrum In Vivo Imaging System, PerkinElmer). The results were shown in FIG. 11.

As shown in FIG. 11, it was confirmed that the fluorescence expression level of the nano-based labeling agent loaded with the methylene blue/indocyanine green fluorescent material composite was significantly higher than that of the control group not treated with the material. The results confirmed that the diagnostic agent loaded with the staining material according to the present invention not only diagnoses the tumor through the intravenous administration method, but also diagnoses the tumor simply by applying the agent to the tissue to distinguish the tumor from the normal tissue.

### Experimental Example 10. Evaluation of the absorption mechanism of a diagnostic agent loaded with staining material into tumor tissue

In order to investigate the absorption mechanism of the diagnostic agent loaded with the staining material into the tumor tissue, the structural change of the tumor was first checked.

FE-SEM (JSM-6700F, JEOL Ltd, Japan) was used to check the morphology of the tissues before and after the diagnostic agent was treated at 40, 100, 500, and 1000 magnifications, respectively. The results are shown in FIG. 12. As shown in FIG. 12, before the tumor was formed, the tissue had micropores, but it was dense and the tissue walls between the pores were wide. However, when a tumor was formed, many large pores were observed, and the tissue walls were definitely formed narrowly compared to normal tissue.

When a diagnostic agent loaded with staining material was applied to a tumor tissue having large pores and a narrow tissue wall, it was observed that the agent was adsorbed and covered not only the inside of the pore but also the tissue wall. Considering the experimental results from a morphological tissue point of view, it was confirmed that the nano-based agent of the present invention may be absorbed and attached due to structural changes caused by tumor formation compared to normal tissues.

Furthermore, the absorption mechanism of nano-based formulations was evaluated in terms of tissue morphological structure as well as the absorption mechanism in living cells. For evaluation, 5×10^4/well of Caco-2 cells were three-dimensionally cultured using 24-well plate-sized trans-wells (BD Biosciences). The cells were treated with a nano-based diagnostic agent to confirm the absorption mechanism. The results are shown in FIG. 13.

As shown in FIG. 13, auto-fluorescence was observed in the cells of the control group that were not treated with any substance, but was significantly lower than that of the group treated with the nano-based diagnostic agent. It was confirmed that when the nano-based diagnostic agent was treated, the strong fluorescence was expressed in the net form. When the image stained using the FITC-phalloidin marker that can stain the action of tight junctions and the image stained by the nano-based diagnostic agent were superimposed, it was found that they match. Thus, it was confirmed that the diagnostic agent loaded with the staining material provided in the present invention may be absorbed through the tight junction.

In summarization of these results, the tumor tissue can be effectively diagnosed by the diagnostic agent loaded with the staining material provided in the present invention through the paracellular pathway, which is the mechanism by which the material is absorbed through the tight junction due to the small particle size and morphological difference of the tumor tissue transformed by inflammation.

### Experimental Example 11. Evaluation of technical diversity by loading various substances into a diagnostic agent for diagnosing tumor tissues through direct application of the agent into tumors

When a tumor is stained, if it is not dependent on the properties of a specific staining material, and a material having a variety of wavelengths is applicable, the staining may be performed by selecting several desired wavelengths. Thus, evaluation was performed to determine whether the tumor was diagnosed by direct application of the nano-based loaded with Chlorin e6 (Ce6, λex = 408 nm) used as a photodynamic treatment in addition to curcumin (λ = 340-534 nm) and methylene blue/indocyanine green fluorescent substance composite (λex = 700, 805 nm) prepared in Experimental Example 1 to an inflammatory colorectal cancer-inducing animal model.

An experiment was conducted for an animal model of inflammatory colorectal cancer using Azoxymethane (AOM)/Dextran sulfate sodium (DSS). 10mg/kg AOM was dissolved in physiological saline (or PBS (pH 7.4)). 5-6 week old C57BL/6 mice were administered at a dose of 5ml/kg of the mixture by intraperitoneal injection. After 1 week of AOM administration, they were administered with the mixture in which 2 w/v. % Dextran sulfate sodium (DSS, Sigma, USA) was dissolved in drinking water for 1 week. After having a rest period of 2 weeks, they were administered with 2% DSS in drinking water for 1 week. They had a rest period of 2 weeks again. The experiment was carried out after breeding for a total of 12 weeks so that an inflammatory tumor could be formed in the large intestine of the animal.

The inflammatory colorectal tumor tissue was treated with a diagnostic agent loaded with staining material having various wavelengths and properties. A fluorescence imaging device (IVIS Spectrum In Vivo Imaging System, PerkinElmer) was used to check the fluorescence change expressed in the tissue. The results are shown in FIGS. 14 to 16, respectively. As shown in each of FIGS. 14 to 16, it was confirmed that the abnormal inflammatory tissue and colon tumor tissue formed by induction of inflammation were effectively stained without depending on the properties of the material loaded in the nano-based agent. The results confirm that the diagnostic agent loaded with the staining material prepared according to the present invention can diagnose tumors formed through cell transplantation, abnormal inflammatory tissues, and tumor tissues formed by induction of inflammation through a conventional intravenous administration method and may be effectively stained by simple application method.

The above has been described with reference to preferred embodiments of the present invention. However, it is understood by those skilled in the art that various modifications and changes can be made in the present invention without departing from the spirit and scope of the present invention as set forth in the following claims.

## Claims

1. A method for preparing nano diagnostic agent capable of selective staining of abnormal inflammatory tissues or tumor tissues, the method comprising: steps of:
forming a first mixture by mixing polysorbate 80, soybean oil, and a water-insoluble staining material;
forming a second mixture by mixing and then heating the first mixture and a triblock copolymer;
forming a nanoplatform solid by cooling the second mixture;
forming a third mixture by mixing a solvent with the nano platform solid;
removing impurities from the third mixture; and
forming a nano-composite by freeze-drying the third mixture from which the impurities are removed.

2. The method of claim 1, wherein the water-insoluble staining material is one staining material selected from the group consisting of curcumin, fluorescein isothiocyanate (FITC), Nile red, Ce6 (Chlorin e6) and PpIX (Protoporphyrin IX).

3. The method of claim 1, wherein the water-insoluble staining material is a water-insoluble staining material composite obtained through ionic bonding by dissolving a cationic first staining material and anionic second staining material in a solvent, respectively, and mixing the same.

4. The method of claim 3, wherein the first staining material is selected from the group consisting of indigo carmine, methylene blue, indocyanine green, toluidine blue and rhodamine B.

5. The method of claim 3, wherein the second staining material is selected from the group consisting of indocyanine green and methylene blue.

6. The method of claim 1, wherein the heating temperature in the step of forming the second mixture is 55 °C to 65 °C.

7. The method of claim 1, wherein the cooling temperature in the step of forming a nanoplatform solid is 0 °C to 10 °C.

8. The method of claim 1, wherein the solvent is tertiary distilled water.

9. A nano diagnostic agent capable of selective staining of inflammatory, the agent including a nano-composite having a micelle structure, wherein the inner core of the micelle structure includes soybean oil and polysorbate 80 in which a water-insoluble staining material is dissolved,
wherein the outer shell of the micelle structure includes a triple block copolymer for forming uniform particles without agglomeration due to precipitation of components included in the inner core in an aqueous solution, and
wherein the nano-composite has an average diameter of 1 nm to 100 nm.

10. A diagnostic agent solution in which the diagnostic agent of claim 9 is dispersed in an injection solution or a buffer solution.
